# EUROPEAN PATENT SPECIFICATION

(11) **EP 2 616 809 B1**
(45) Date of publication and mention of the grant of the patent: **05.04.2017**
(21) Application number: 11825980.3
(22) Date of filing: 15.09.2011
(51) Int. Cl.: C07K 1/22, G01N 33/53, C08B 37/00, C08H 1/00, G01N 33/68

(54) **ISOLATION AND DEGLYCOSYLATION OF GLYCOPROTEINS**
ISOLATION UND DEGLYKOSYLIERUNG VON GLYKOPROTEINEN
ISOLATION ET DÉGLYCOSYLATION DES GLYCOPROTÉINES

(30) Priority: 17.09.2010 US 384185 P
(43) Date of publication of application: 24.07.2013
(73) Proprietor: Prozyme, Inc., Hayward, CA 94545 (US)
(72) Inventor: FULTON, Scott P., Madison, WI 53717 (US); Murphy, Steven M., Madison, WI 53717 (US)
(74) Representative: Secerna LLP
(86) International application number: PCT/US2011/051840
(87) International publication number: WO 2012/037407

(56) References cited:
- WO-A1-95/15969
- WO-A1-2009/100155
- US-A1- 2007 269 895
- US-A1- 2010 190 146
- US-A1- 2010 190 146
- US-B1- 8 198 063
- J. GEYER ET AL: "Cloning and Functional Characterization of Human Sodium-dependent Organic Anion Transporter (SLC10A6)", JOURNAL OF BIOLOGICAL CHEMISTRY, vol. 282, no. 27, 1 July 2007 (2007-07-01) , pages 19728-19741, XP055081428, ISSN: 0021-9258, DOI: 10.1074/jbc.M702663200
- PAPAC D I ET AL: "A HIGH-THROUGHPUT MICROSCALE METHOD TO RELEASE N-LINKED OLIGOSACCHARIDES FROM GLYCOPROTEINS FOR MATRIX-ASSISTED LASER DESORPTION/IONIZATION TIME-OF-FLIGHT MASS SPECTROMETRIC ANALYSIS", GLYCOBIOLOGY, OXFORD UNIVERSITY PRESS, US, vol. 8, no. 5, 1 May 1998 (1998-05-01), pages 445-454, XP009016608, ISSN: 0959-6658, DOI: 10.1093/GLYCOB/8.5.445
- ALBERT S. B. EDGE: "Deglycosylation of glycoproteins with trifluoromethanesulphonic acid: elucidation of molecular structure and function", BIOCHEMICAL JOURNAL, vol. 376, no. 2, 1 December 2003 (2003-12-01), pages 339-350, XP055104408, ISSN: 0264-6021, DOI: 10.1042/BJ20030673
- MAGGIE A. BYNUM ET AL: "Characterization of IgG N-Glycans Employing a Microfluidic Chip that Integrates Glycan Cleavage, Sample Purification, LC Separation, and MS Detection", ANALYTICAL CHEMISTRY, vol. 81, no. 21, 1 November 2009 (2009-11-01), pages 8818-8825, XP055000606, ISSN: 0003-2700, DOI: 10.1021/ac901326u
- ANGELA HAUSINGER ET AL: "Two synpatic vesicle proteins of 25 kDa: A comparison of the molecular properties and tissue distribution of svp25 and o-rab3", NEUROCHEMISTRY INTERNATIONAL, vol. 28, no. 3, 1 March 1996 (1996-03-01), pages 251-258, XP055104584, ISSN: 0197-0186, DOI: 10.1016/0197-0186(95)00087-9
- DAKOUR J ET AL: "Detection and isolation of oligosaccharides with Le<a> and Le<b> blood group activities by affinity chromatography using monoclonal antibodies", ARCHIVES OF BIOCHEMISTRY AND BIOPHYSICS, ACADEMIC PRESS, US, vol. 264, no. 1, 1 July 1988 (1988-07-01), pages 203-213, XP024762771, ISSN: 0003-9861, DOI: 10.1016/0003-9861(88)90586-3 [retrieved on 1988-07-01]
- LIWEI CAO ET AL: "N-Glycosylation Site Analysis of Proteins from Saccharomyces cerevisiae by Using Hydrophilic Interaction Liquid Chromatography-Based Enrichment, Parallel Deglycosylation, and Mass Spectrometry", JOURNAL OF PROTEOME RESEARCH, 1 February 2014 (2014-02-01), XP055104562, ISSN: 1535-3893, DOI: 10.1021/pr401049e
- GEYER ET AL.: 'Cloning and functional characterization of human sodium-dependent organic anion transporter (SLC10A6).' J. BIOL. CHEM. vol. 282, no. 27, 06 July 2007, pages 19728 - 19741, XP055081428
- YU J.: 'Expression and Characterization of E-LecEGF for Structural Study and Assay Development.' PHD THESIS, [Online] 2009, UNIVERSITY OF BASEL, FACULTY OF SCIENCE, pages FP - 169, XP055081430 Retrieved from the Internet: <URL:http://edoc.unibas.ch/954> [retrieved on 2012-01-09]
- LI ET AL.: 'Hydrophobic interaction chromatography correctly refolding proteins assisted by glycerol and urea gradients.' J. CHROMATOGR. A. vol. 1061, no. 2, 24 December 2004, pages 193 - 199, XP004990653

## Description

### CROSS-REFERENCE TO RELATED APPLICATIONS

This application claims the benefit of U.S. Provisional Application No. 61/384,185, filed September 17, 2010.

### STATEMENT OF FEDERAL FUNDING

Not applicable.

### BACKGROUND OF THE INVENTION

This invention relates to the field of analysis of glycosylation patterns on glycoproteins.

Many of the proteins produced by eukaryotic cells are modified after translation by the addition of covalently-linked, linear or branched chains of carbohydrates. These protein-carbohydrate conjugates are referred to as glycoproteins; the point at which the carbohydrate is attached is referred to as a glycosylation site. Attached polysaccharides or oligosaccharides are referred to as glycans. A wide range of glycans are found on the different glycosylation sites of particular glycoproteins. The particular pattern of glycans on a particular glycoprotein is determined by the specific cell line that produced the protein and the conditions under which the cells were grown.

Since the glycans conjugated to a protein can affect characteristics critical to its function, including pharmacokinetics, stability, bioactivity, or immunogenicity, it is important in many uses to determine which glycans are present. Thus, the ability to remove some or all of the glycans from a protein and to analyze the glycans or the protein, or both, to determine their composition or compositions is useful for determining whether a protein will have a desired effect. For example, the Food and Drug Administration requires characterization of carbohydrates attached to biologics (such as therapeutic glycoproteins and vaccines) to show composition of matter and consistency of manufacture, resulting in a need for extensive characterization of the product. Analysis of the profile of the released carbohydrates is also important for quality control in the production of recombinant proteins, in which a change in carbohydrate profile may indicate stress in the system, signaling conditions that may require a commercial-scale fermenter of expensive protein to be discarded.

The techniques used to analyze glycans are complex, cumbersome and time-consuming. The glycans must be released or removed from the protein, a process known as "deglycosylation", before analysis can be performed. Further, the samples to be analyzed typically include cell lysates, cell culture supernatants, and clinical plasma or serum samples, which may contain a multitude of glycoproteins in addition to the one of interest. Thus, companies wishing to obtain analysis of the carbohydrates attached to a particular glycoprotein, such as an antibody intended for therapeutic use in humans, often have first to perform a number of steps to isolate the target glycoprotein from others in the raw sample.

Only a subset of proteins can be deglycosylated under native, or non-denaturing, conditions, in which the protein is simply mixed with an enzyme that will release from the protein the glycans conjugated to the protein. These methods have the advantage of mild conditions and simple clean up, but often result in incomplete release of glycans. For most proteins being deglycosylated by enzymatic digestion, however, the secondary and tertiary structures of the proteins do not permit access of the enzyme to the carbohydrates unless the protein is first denatured to alter those structures. Typical protocols for denaturing involve the use of detergents and reducing agents, and an overnight incubation. For example, these protocols typically adding to the glycoprotein a reducing agent such as beta-mercaptoethanol, an anionic detergent, such as sodium dodecyl (lauryl) sulfate, a non-ionic detergent, such as octylphenolpoly(ethyleneglycolether), and a deglycosylating enzyme, and incubating the resulting mixture for 16 hours at 37 °C. Once the protein is deglycosylated, the glycans are removed and, usually, are labeled. These protocols are effective and largely independent of the protein (that is, they can be used on most proteins), but are harsh, typically use detergents, which must be removed before some analytical processes can be conducted, and have more clean-up steps.

In commercial cell culture settings, the length of sample processing, typically at least a day, when these standard deglycosylation methods are used reduces the possibility of using carbohydrate analysis as a marker for rapid, in-process analysis and as a tool for control of process variables, such as stress during the cell culture process. The use of detergents adds steps and time since any detergent remaining with the released glycans can interfere with the results of mass spectrometry or other techniques used to analyze the carbohydrate profile

Deglycosylation, in turn, is achieved by one of two methods, enzymatic digestion or chemical release. In enzymatic digestion, for example, N-glycans (glycans linked to the protein through amide groups of asparagine residues) are released from glycoproteins by enzymatic cleavage with PNGase F (Peptide-N4-(acetyl-ß-glucosaminyl)-asparagine amidase, EC 3.5.1.52) or endoglycosidases such as endo-alpha-N-acetyl-galactosaminidase, Endoglycosidase F1, Endoglycosidase F2, Endoglycosidase F3, or Endoglycosidase H. The glycans are then typically treated to label their free-reducing terminus with a fluorescent dye, excess label in removed, and the labeled glycans analyzed by methods such as high performance liquid chromatography (HPLC), capillary electrophoresis (CE), carbohydrate gel electrophoresis, or mass spectrometry. Both the enzymatic and the chemical deglycosylation procedures encompass multiple steps, extended incubation times, and clean-up steps prior to analysis of the released glycans. The release and analysis of the glycans typically takes up to 3 days.

Papac et al., "A high-throughput microscale method to release N-linked oligosaccharides from glycoproteins for matrix-assisted laser desorption/ionization time-of-flight mass spectrometric analysis," Glycobiology (8(5):445-454 (1998), discloses the deglycosylation of low microgram quantities of glycoproteins by immobilizing them on 96-well plates lined with a high protein-binding membrane, polyvinylidene difluoride (PVDF), releasing the glycans with PNGase F, and detecting the released glycans by matrix-assisted laser desorption/ionization time-of-flight (MALDI-TOF) mass spectrometry. WO95/15969 relates to method of preparing sugar derivatives either in isolation or from glycopeptides or glycoproteins. A process described in WO95/15969 chemically immobilizes glycoproteins on a solid support and then subjects the glycoprotein to having its sugars removed by Edman degradation. Geyer et al (Journal of Biological Chemistry, vol. 282, no. 27 (1 July 2007), pgs. 19728-19741 describes the cloning and characterization of a human glycoprotein, SOAT. The method of isolating SOAT comprises the use of immobilized Protein A.

There remains a need for methods that can be performed rapidly, under relatively mild conditions, that reduce handling and clean up steps, that are suitable for use with multiple analytic methods, and that improve the amount and types of glycans released. Surprisingly, the present invention meets these and other needs.

### BRIEF SUMMARY OF THE INVENTION

The present invention provides faster, more convenient ways of isolating and deglycosating glycoproteins, and permits ready quantitation of both the glycoproteins and of the glycans attached to them. The present invention is as set forth in the claims.

In a first group of embodiments, the specification provides methods of releasing glycans from a target glycoprotein in a sample (such as a biological sample), comprising performing the following steps in the following order: (a) adding a sample to a solid phase support comprising (i) a solid phase and (ii) an affinity ligand which binds to the target glycoprotein, which molecule is immobilized on the solid phase, thereby binding the target glycoprotein to the molecule on the solid phase; (b) washing the solid phase with a buffer to wash away any unbound glycoprotein; and, (c) contacting the bound target glycoprotein on the solid phase with an enzyme that catalyzes the release of glycans from glycoproteins, thereby releasing the glycans from the target glycoprotein. In some embodiments, the target glycoprotein is an antibody. In some embodiments, the affinity ligand is Protein A. In some embodiments, the affinity ligand is a deglycosylated antibody or binding fragment thereof. In some embodiments, the buffer comprises a detergent. In some embodiments, the detergent is a non-ionic detergent. In some embodiments, the enzyme is selected from the group consisting of an amidase and an endoglycosidase. In some embodiments, the amidase is PNGase F. In some embodiments, the solid phase support is a porous chromatography media. In some embodiments, the media is in a packed bed. In some embodiments, the bound target glycoprotein is eluted from said solid phase support and quantitated. In some embodiments, the quantitation is by measuring absorbance of ultraviolet light at approximately 280 nm. In some embodiments, the released glycans are quantitated. In some embodiments, the solid phase support is a microcolumn or microcartridge. In some embodiments, the method optionally includes step (a'), adding a blocking reagent, between step (a) and step (b).

In a second group of embodiments, the specification discloses methods of releasing glycans from a target glycoprotein in a sample (such as a biological sample), the method comprising performing the following steps in the following order: (a) adding the biological sample to a solid phase support comprising (i) a solid phase and (ii) an affinity ligand, which affinity ligand is immobilized on the solid phase, thereby binding the target glycoprotein to the molecule on the solid phase; (b) washing the bound target glycoprotein with a wash buffer to wash away any unbound glycoprotein; (c) releasing the bound target glycoprotein to free the target glycoprotein, and eluting the target glycoprotein from the solid phase support; (d) optionally, quantitating the amount of the target glycoprotein eluted from the solid phase support; and (e) contacting the target glycoprotein with an enzyme that catalyzes the release of glycans from glycoproteins, thereby releasing the aid glycans from the target glycoprotein. In some embodiments, the method further comprises between steps (d) and (e), step (d') adding the target glycoprotein to a solid phase support having a hydrophobic surface, thereby binding the target glycoprotein to the hydrophobic surface; and, optionally, step (d"), adding a blocking agent to block further binding interactions with the hydrophobic surface. In some embodiments, the target glycoprotein is an antibody. In some embodiments, the affinity ligand is Protein A. In some embodiments, the affinity ligand is a deglycosylated antibody or binding fragment thereof. In some embodiments, the enzyme is selected from the group consisting of an amidase and an endoglycosidase. In some embodiments, the amidase is PNGase F. In some embodiments, the optional quantitation is by measuring absorbance of ultraviolet light at approximately 280 nm. In some embodiments, the released glycans are quantitated. In some embodiments, the solid phase support is a microcolumn or microcartridge.

### BRIEF DESCRIPTION OF THE DRAWINGS

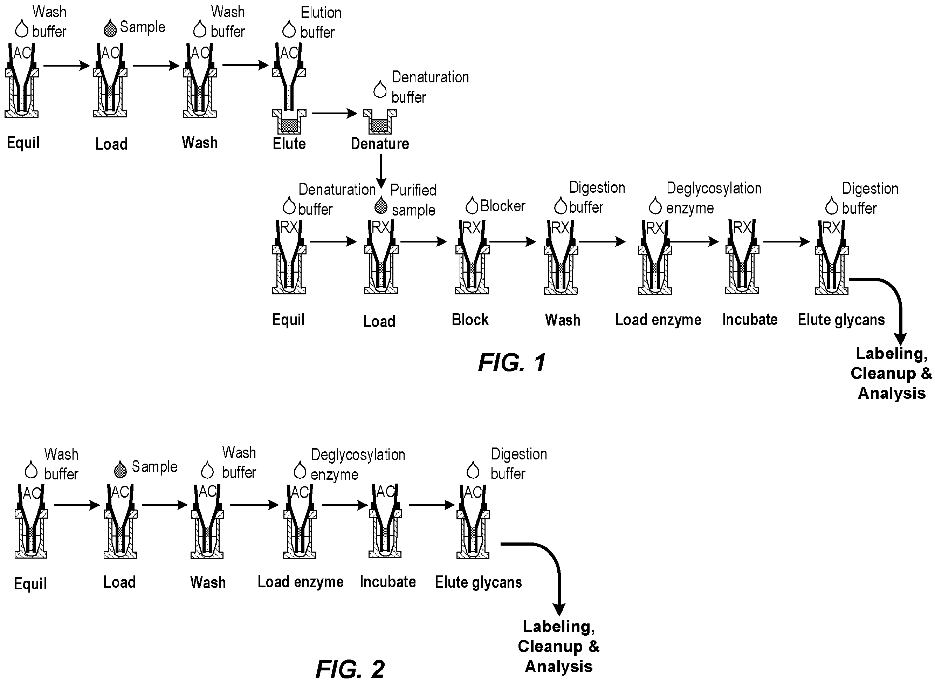

**Figure 1.** Figure 1 shows a procedure in which a sample is added to an affinity chromatography column ("AC"), captured and purified from the initial sample by an affinity ligand, eluted, denatured, loaded into a reaction vessel ("RX"), and deglycosylated by an amidase or endoglycosidase. The released glycans are then available for analysis. "Equil" is short for "equilibrate."
**Figure 2.** Figure 2 shows an *"in situ* deglycosylation" embodiment of the present invention. The sample is added to an affinity chromatography column ("AC"), the target glycoprotein is captured and purified from the initial sample by an affinity ligand, washed, and deglycosylated *in situ* by an amidase or endoglycosidase. The released glycans are then available for labeling and analysis. "Equil" is short for "equilibrate."

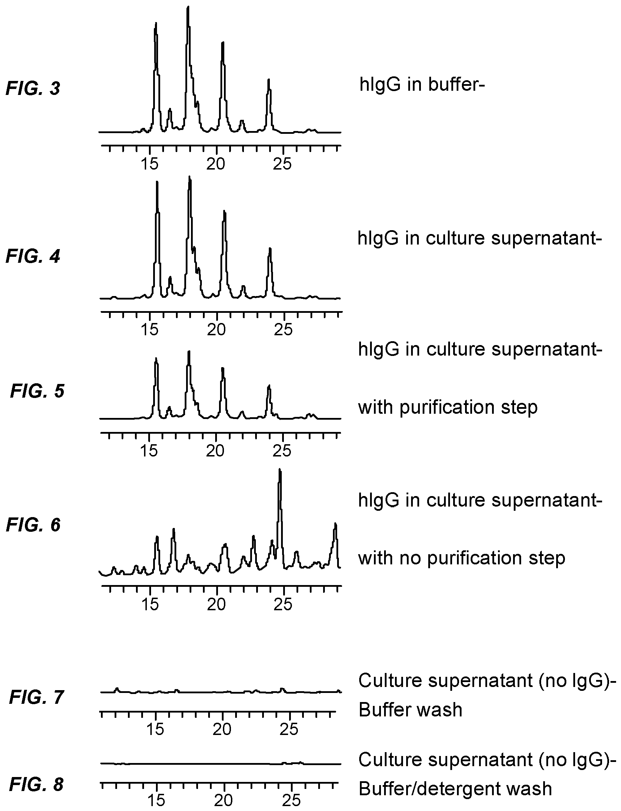

**Figure 3.** Figure 3 shows a HPLC chromatogram of the glycan profile resulting from using the *"in situ* deglycosylation" method of the present invention to analyze the glycans of polyclonal human IgG (hIgG) in PBS buffer. Vertical axis: amount of glycan present. Horizontal axis: time.
**Figure 4.** Figure 4 shows a HPLC chromatogram of the glycan profile resulting from using the "*in situ* deglycosylation" method of the present invention to analyze the glycans of polyclonal human IgG (hIgG) spiked into a cell culture supernatant. Vertical axis: amount of glycan present. Horizontal axis: time.
**Figure 5.** Figure 5 shows a HPLC chromatogram of the glycan profile resulting from using analysis of the glycans of polyclonal human IgG (hIgG) spiked into cell culture supernatant using separate cartridges for the initial purification step and for deglycosylation, as shown in Figure 1. Vertical axis: amount of glycan present. Horizontal axis: time.
**Figure 6.** Figure 6 shows a HPLC chromatogram of the glycan profile resulting from analyzing the glycans of polyclonal human IgG (hIgG) in cell culture supernatant without first isolating the hIgG by binding it to an affinity ligand. Vertical axis: amount of glycan present. Horizontal axis: time.
**Figure 7.** Figure 7 shows a HPLC chromatogram of the glycan profile resulting from analyzing the glycans of the cell culture supernatant without addition of hIgG. Vertical axis: amount of glycan present. Horizontal axis: time.
**Figure 8.** Figure 8 shows a HPLC chromatogram of the glycan profile resulting from analyzing the glycans of a polyclonal human IgG (hIgG) spiked into cell culture supernatant using separate cartridges for the initial purification step and for deglycosylation, but with a non-ionic detergent added to the wash buffer.

### DETAILED DESCRIPTION

As noted in the Background, analyzing the carbohydrates attached to proteins is important to understanding the pharmacokinetics, immunogenicity, and potential effectiveness of the proteins for their intended use. These analyses are, however, made more difficult by the fact that the initial samples, such as patient sera or supernatants from cultures of recombinant cells, often contain large amounts of glycoproteins other than the one of interest (the one of interest is sometimes referred to herein as the "target glycoprotein"). Currently, these contaminating glycoproteins are typically removed by the group which produces the target glycoprotein, and then provided to a glycan analysis group, which conducts an elaborate series of 20 or more denaturing, wash, reduction, labeling and analysis steps.

Surprisingly, the present invention provides methods of releasing glycans from glycoproteins that not only involves sharply reduces the number of steps than current techniques, but also reduces the amount of enzyme and other reagents needed. Even more surprisingly, the methods of the present invention permit the quantitative recovery of both the target glycoprotein and of the glycans released from the protein in a single workflow and without knowing the amount of glycoprotein present in the initial patient sera or culture supernatant sample. Further, the methods result in a surprisingly high degree of reproducibility.

The methods of the present invention combine a selective purification and immobilization step that facilitates the isolation of the target glycoprotein from an initial sample, which may be a biological sample such as a cell culture supernatant, cell lysate or patient serum sample, onto a solid support. As further explained below, in one embodiment, the purified, immobilized glycoprotein is then eluted from the solid support, denatured, immobilized on a second solid support and incubated with enzymes that release the glycans from the glycoprotein. If desired, the eluted protein can be eluted into a container, such as a well on a conventional microtiter plate, and the amount of protein read on a conventional plate reader before denaturation and deglycosylation. Persons of skill are aware that there are a number of methods for quantitating the amount of protein in a sample, but one convenient method is to use a spectrophotometer to determine the amount of UV absorbance near 280 nm. The amount of protein present in the eluted sample, and the amount of glycans released from the eluted protein, can then be compared to, for example, previous or future samples from the same patient or the same culture. In the case of a culture supernatant, for example, a change in the relative amounts of protein and released glycan indicates that the culture conditions are changing and should be checked.

In a more preferred embodiment, the purified, immobilized glycoprotein is deglycosylated while still immobilized on the solid support, eliminating even more of the steps used in current protocols. The eluted glycans are quantitated, and the target glycoprotein (now deglycosylated) can be released from the solid support and quantitated as described above. Figure 2 shows an exemplary in situ embodiment of the present invention.

Since the inventive methods permit purification of a glycoprotein of interest directly from a mixture of other proteins and glycoproteins, samples containing the target glycoprotein, and the target glycoprotein itself, other isolation procedures, such as gel electrophoresis (e.g., polyacrylamide gel electrophoresis, or "PAGE") or immunoblotting, such as western blotting, should not be performed before subjecting the sample or glycoprotein to the inventive methods. Further, since increased speed and a reduced number of steps are advantages of the present invention, neither the sample nor the target glycoprotein should be subjected to gel electrophoresis or immunoblotting procedures in between the various steps of the inventive methods (for example, immunoblotting should not be performed on the sample or target glycoprotein between the immobilization and deglycosylation steps of the inventive methods).

In the present invention, the purification and isolation step is performed by subjecting the initial sample to affinity chromatography. Since columns for affinity chromatography are widely available, the use of an affinity chromatography column is preferred and the discussion below will be stated in terms of use of a column, although other solid supports can be used. For example, in addition to the packed bed typically used in packed bed columns, the affinity chromatography could be by batch processing with either centrifugal or filtration-based separation of the solid phase from the liquid phase or membranes with immobilized affinity ligands. To emphasize that the solid phase support may be in a configuration other than a column, it may also be referred to herein generically as a "solid phase support."

The inventive methods may be practiced with a variety of conventional solid phase extraction devices, such as small chromatography columns, spin columns, or pipette tips in which resin or other solid phase medium is held in place. In preferred embodiments, the column is one adapted for use with microliter volumes of reagents (such a column is sometimes referred to as a "microcartridge"), to facilitate deglycosylation and analysis of glycoproteins that may be available only in very limited quantities or that are particularly expensive. One microcartridge suitable for use in the invention is, for example, the AssayMAP® microcartridge (BioSystem Development, LLC, Madison, WI), which employs a 5 µL bed. The column is typically packed with a solid or stationary phase or medium (which may collectively be referred to as the "solid phase"), as is done for conventional affinity chromatography. The AssayMAP® microcartridge, for example, can be packed with a variety of beaded resins to serve as the solid phase for use in the inventive methods. Figures 1 and 2 illustrate and compare deglycosylation methods using AssayMAP® microcartridges as exemplar solid phase extraction devices. In both Figures 1 and 2, liquids are pipetted into the "cup" of the microcartridge, delivering them to the solid phase (shown by stippling) packed in the pipette-like tip of the microcartridge. Particulate solid phases like the ones depicted in the Figures are typically held in place by filters (not shown) at either end of the pipette-like tip. The slightly darker stippling shown at the top of the pipette-like tip in some of the steps represents glycoprotein immobilized where it first contacts the solid phase.

While the AssayMAP® microcartridge employs a 5 µL bed, other volumes can be used. For example, in some embodiments, the volumes are between 3 and 50 µL, in some embodiments are between 3 and 40 µL, in still other embodiments, are between 3 and 30 µL, and preferably are between 5 and about 25 µL, and in some embodiments is about 10 to about 20 µL, with "about" meaning 3 µL on either side of the stated quantity. Five µL generally provides enough glycans for analysis using current techniques, while minimizing the amount of what may be a very limited amount of target glycoprotein available for use in the analysis. If the amount of target glycoprotein available is not so limited or so expensive that only a few µL can be made available for testing, a bed volume such as about 10 or about 15 µL may be preferred, with "about" meaning 3 µL on either side of the stated quantity.

In the inventive methods, the solid phase further comprises a molecule chosen for its specific biological interaction with the target glycoprotein. For example, the solid phase may have bound or attached to it a ligand specific for the glycoprotein of interest or a lectin specific for a desired glycan. For convenience of reference, the molecule selected for its specific biological interaction with the glycoprotein of interest will be referred to herein as the "affinity ligand." To illustrate with reference to Figure 2, for example, in the inventive methods, the solid phase shown by stippling in the pipette-like tip of the microcartridge comprises an affinity ligand specific for the target glycoprotein.

Conveniently, the affinity ligand is covalently linked to the solid support by, for example any of a variety of chemistries, such as N-Hydroxysuccinimide (NHS) esters, epoxide, aldehyde, or cyanogen bromide, to a solid phase. Such conjugation chemistries are well-known in the art, as exemplified in Hermanson, G.T., Bioconjugate Techniques, Academic Press (Amsterdam, the Netherlands, 2nd Ed. 2008) and Wong, S., Chemistry of Protein Conjugation and Cross-Linking, CRC Press (Boca Raton, FL, 1991). The solid phase is selected to be one having very low non-specific adsorption for proteins. Conventional examples of such solid phases are well known in the art and include agarose beads, acrylic beads, or polystyrene beads with a polymeric hydrophilic coating. Since the non-target glycoproteins do not interact with the affinity ligand, they can be readily washed away. The media chosen for affinity chromatography is usually selected to minimize non-specific binding of target reagents.

In a first group of embodiments, once the target glycoprotein is isolated by binding to the affinity ligand on the solid phase, the non-target glycoproteins are washed away in a wash step. We have found that the presence of a detergent in the wash buffer reduces the background by a factor of at least 5 and as much as 10. Accordingly, the wash buffer preferably contains a detergent. Following the wash, a deglycosylation enzyme is added to the solid phase containing the bound target glycoprotein, and the solid phase is incubated to permit the enzymatic release of glycans from the target glycoprotein. The glycans are then eluted and recovered for labeling and analysis.

This group of embodiments greatly simplifies the glycan preparation process by eliminating numerous steps of elution, denaturation, rewetting, equilibration, washing, and blocking, reducing the overall time required for the procedure and reducing the chance of sample loss. This procedure also facilitates the collection of the released glycans in a minimal volume at a relatively high concentration, which facilitates the downstream labeling and cleanup steps. For ease of reference, this embodiment is sometimes referred to herein as *"in situ* deglycosylation."

In our *in situ* deglycosylation embodiments, no denaturation step is included, as the binding of the target glycoprotein to the affinity ligand would likely not survive the resulting conformational changes. Not all glycoproteins, however, need to be denatured to recover most or all of the attached glycans. The *in situ* deglycosylation method is, in fact, particularly useful in the deglycosylation of antibodies, which typically do not need to be denatured to be fully deglycosylated. This is fortunate because, as has already been noted, antibodies constitute the most important group of biologic therapeutics for which glycan characterization is needed.

In a second group of embodiments, after the isolated target glycoprotein is bound to the affinity ligand and washed to remove the unbound, non-target glycoproteins, the target glycoprotein is eluted from the column with, for example, low pH buffers. Methods for eluting antibodies and other glycoproteins from columns are well known in the art and various elution buffers are commercially available for this purpose. If desired, the target glycoprotein can then be denatured and deglycosylated in solution as in conventional techniques. In preferred embodiments, however, the now-purified, eluted target glycoprotein is first denatured by addition of a denaturing buffer, after which it is loaded onto a hydrophobic surface, such as a column, which is preferably first wet and then equilibrated with the denaturing buffer. The hydrophobic surface can be any conventional protein-binding material. The loading of the sample immobilizes the now denatured target glycoprotein on the hydrophobic surface. A blocking agent can be added to prevent non-specific binding of enzyme to the hydrophobic surface, and the column is washed with a buffer compatible with the endoglycosidase or other enzyme to be used for deglycosylation. The deglycosylation enzyme is then added and the hydrophobic surface is incubated to release the glycans, which are then eluted from the hydrophobic surface and recovered for further labeling, cleanup and analysis. Because the target glycoprotein is immobilized on the hydrophobic surface rather than free in solution, application of the enzyme to the column results in a high localized concentration, permitting the use of a small amount of enzyme. The high localized concentration also decreases the time necessary to achieve hydrolysis of the N-linked glycans on the target glycoprotein, and permits quantitative recovery of the released glycans.

### Materials for use in the solid phase

The solid phase used for the affinity chromatography methods herein can be made from any material on which a highly selective affinity ligand can be immobilized, and which offers very low non-specific adsorption of non-target proteins (including non-target glycoproteins). Typical affinity chromatography media are generally suitable for this application. These media include agarose or other polysaccharides, polyacrylics, polystyrene or other synthetic polymers (potentially including hydrophilic surface coatings), coated silica, zirconia or other inorganic porous materials and various composites of the above. The media may be in the form of a packed bed of beads, a monolith or a membrane. The affinity chromatography may be performed using centrifugation, gravity, positive pressure, pumping or vacuum to drive liquids employed in the methods through the solid phase.

### Affinity ligands

The molecule chosen for its specific biological interaction with the glycoprotein of interest is desirably highly selective for the target glycoprotein and preferably does not bind to other glycoproteins which may be present in the sample to be introduced. To avoid release of glycans that might confound the analysis, the ligand is preferably not a glycoprotein or, if it is, has had any N-glycan groups present on the ligand removed prior to its use in the methods of the invention. Further, in the embodiments in which the deglycosylation is performed while the target glycoprotein is bound to the affinity ligand, the affinity ligand must be capable of retaining the target glycoprotein under the conditions required for the deglycosylation reaction. Finally, the affinity ligand's binding site to the target glycoprotein is preferably far enough removed from the site at which the glycans are attached to the glycoprotein so that the enzyme used for deglycosylation has access to the glycans while the glycoprotein is bound to the affinity ligand. In some embodiments, the affinity ligand is a monoclonal antibody specific for the target glycoprotein, which antibody has already been deglycosylated so that it does not have glycans that could confound or interfere with the analysis of glycans on the target glycoprotein. In some embodiments in which the target glycoprotein is an antibody, the affinity ligand is an anti-idiotypic antibody which is specific for the target glycoprotein. In other embodiments, the affinity ligand is an antibody specific for the glycoprotein of interest. Antibodies can be generated against any particular target glycoprotein using conventional techniques. The antibodies so generated can be tested to see if they bind too close to a glycosylation site on the target glycoprotein for use in the methods of the invention by conventional epitope mapping or by, for example, comparing the amount of glycan released from a given amount of target glycoprotein bound to the antibody so generated and that released from the same amount of target glycoprotein bound instead to Protein A.

In some embodiments, such as the glycoanalysis of recombinant antibodies produced by a culture of transfected cells, the target glycoprotein will be of a particular category or type (in this example, an immunoglobulin) and will be expected to be the only glycoprotein of its type present in the initial sample. For example, therapeutic antibodies are often expressed in cultures of recombinant Chinese hamster ovary (CHO) cells. Ovary cells, of course, do not produce antibodies as part of their normal repertoire, so the only antibodies expected to be present in the culture supernatant would be the expressed, recombinant antibody. Thus, in these applications, the affinity ligand need only selectively bind glycoproteins of the target type, such as immunoglobulins, to be able to purify the target glycoprotein from any number of other glycoproteins in the initial sample.

In a preferred group of embodiments, the affinity ligand is Protein A. Protein A, originally isolated as a protein from the cell wall of *Staphylococcus aureus*, is not glycosylated and is commercially available from a number of sources, including Sigma-Aldrich (St. Louis, MO, catalog no. P6031), Pierce Protein Research Products (Thermo Fisher Scientific, Rockford, IL, catalog no. 20333) and Santa Cruz Biotechnology, Inc. (Santa Cruz, CA, catalog no. sc-2001). It is useful for binding immunoglobulins, including the secreted immunoglobulins known as antibodies, and is particularly useful for binding IgG antibodies. Conveniently, Protein A binds antibodies on the Fc portion at a site well removed from the N-glycosylation site(s), which tend to be near the hinge region, and thus does not interfere with the activity of PNGase F and endoglycosidases. Further, Protein A can release the immunoglobulins for analysis. Thus, Protein A is particularly valuable for use as the affinity ligand for isolating antibodies from supernatants of cultures of recombinant transfected cells, such as CHO or yeast cells.

In some embodiments, the affinity ligand is Protein G or Protein L, which are commercially available from a number of sources, including Thermo Fisher Scientific and Sigma-Aldrich. The binding affinities of these ligands is well known and the selection of one as a ligand for a glycoprotein of interest is considered routine for persons of skill in the art. It is noted that Protein G, like Protein A, binds the Fc portion of mammalian IgG, but does so with higher affinity and can bind IgG from a wider variety of sources. Protein L, by contrast, binds kappa light chains, and is suitable for purifying antibodies from species whose antibodies contain kappa light chains, but not from goats, sheeps, horses, or cows, whose Igs do not contain kappa chains. Protein G, Protein L, and any other candidate for use as an affinity ligand, can be readily tested for its suitability for use with any particular target glycoprotein by using the Protein G, Protein L, or other candidate affinity ligand, in the methods of the invention with a known amount of a target glycoprotein having a known quantity of glycans, deglycosylating the target glycoprotein with a deglycosylation enzyme of choice, and determining if the quantity of glycans recovered is within a desired range of the quantity of glycans known to be present. If so, it indicates that the candidate affinity ligand binds the target glycoprotein in a manner that renders the glycans accessible to a particular deglycosylation enzyme, and is suitable for use with that target glycoprotein.

In another preferred group of embodiments, the affinity ligand is a Camelid-derived single domain antibody fragment that specifically binds the antibody or other glycoprotein of interest. Such antibody fragments are sometimes referred to as "VHHs". One commercial source of such fragments is BAC BV (Naarden, the Netherlands). BAC's CaptureSelect® Antibody Toolbox provides, for example, the "Fab kappa affinity matrix", that can selectively bind human antibodies with kappa light chains, the "Fab lambda affinity matrix", that can bind human antibodies with lambda light chains, the "Human Fc affinity matrix", that can bind any subclass of human IgG, and the "Multi Species affinity matrix", that can bind IgGs from multiple species. One preferred BAC BV antibody fragment, resold by GE Healthcare, is IgSelect (GE Healthcare Bio-Sciences Corp., Piscataway, NJ, catalog no. 28-4113-01), a 14 kD single chain antibody fragment designed for selectivity towards the Fc portion of human IgG and is particularly useful for binding IgG₃, which is not readily bound by protein A. This ligand can be used alone or in combination with Protein A. Alternatively, the practitioner can generate VHHs for an antigen of interest. Conveniently, this is done by immunizing a camelid, such as a llama, with the antigen and panning the resulting library of antibodies for binders to the antigen, as taught in, e.g., Goldman et al., Anal Chem, 80(22):8583-91 (2008); Conway et al., PLoS One 5(1):e8818. doi:10.1371/journal.pone.0008818 (2010); and Hultberg et al., PLoS One 6(4):e17665. Doi:10.1371/journal.pone.0017665.

### Washing conditions

Washing conditions for the affinity column are important for the methods of the invention. In normal affinity chromatography, the target is first bound to the column, non-binding proteins are washed away and the target is selectively eluted. Some non-specifically bound proteins may remain bound even during elution, and are nonetheless separated in normal chromatography. In the *in situ* glycosylation embodiments of the present invention, these non-specifically bound proteins will be present during the enzyme reaction and, if any are glycoproteins, they will contaminate the final result. Thus extra measures should be taken to remove as much of these non-specifically bound proteins as possible.

Typically, the wash conditions selected are chosen because they will not break the bind between the affinity ligand and the target glycoprotein. The studies underlying the present invention revealed that washing the bound glycoprotein with a detergent prior to deglycosylation reduced the background from non-targeted glycoproteins by a factor of 5, or even of 10. The detergent can be an anionic detergent, such as sodium dodecyl (lauryl) sulfate, or a non-ionic detergent, such as octylphenolpoly(ethyleneglycolether). In some preferred embodiments, the detergent is the non-ionic detergent commonly known as NP-40 (nonyl phenoxypolyethoxylethanol) or Tergitol® type NP-40

In preferred embodiments, the wash with buffer containing a detergent is followed by a wash with a detergent-free buffer to remove any detergent that may have remained behind. The wash can be with any standard laboratory buffer that the literature indicates is compatible with the deglycosylation enzyme to be used. For use with the exemplar deglycosylation enzyme PNGase F, for example, in some embodiments, the buffer is tris hydrochloride, in others, phosphate buffered saline, in others, it is ammonium bicarbonate, and in still others, it is ammonium formate. The concentration of the buffer is typically 5 to 100 millimolar, in some embodiments, 5 to 50 millimolar, in others 5 to 25 millimolar, in others 5-15 millimolar, and in others 5-10 millimolar, while in yet others, it is 5 millimolar.

### EXAMPLES

A series of studies were conducted analyzing N-glycans on human IgG. Over half of the current biopharmaceuticals under development are monoclonal IgG antibodies, and the glycans on virtually all antibodies are N-glycans. In the studies reported below, Protein A was used as the affinity ligand for both analytical and preparative purification of IgG.

In the studies reported below, polyclonal human ("h") IgG purified from human plasma was used. The sample was placed into either standard phosphate buffered saline ("PBS") buffer or a Chinese Hamster Ovary ("CHO") cell culture supernatant which did not contain hIgG. Enzymatic deglycosylation was performed using the exemplar deglycosylation enzyme PNGase F, and the released N-glycans were labeled with InstantAB^{™} dye (ProZyme, Inc., Hayward, CA). Excess dye was removed using hydrophilic interaction solid phase extraction (reagents and cartridges from the GlykoPrep™ N-Glycan kit, ProZyme, Inc.) The samples were analyzed using a hydrophilic interaction HPLC column (GlykoSep^{™} N Plus, ProZyme, Inc.) with a reverse acetonitrile gradient, using a fluorescence detector.

### Example 1.

In this study, hIgG (25 µg) in PBS was loaded on an AssayMAP^{™} PA cartridge (BioSystem Development, LLC., Madison, WI), which contains a 5-µL bed volume of an immobilized Protein A resin, and is operated as a spin column. After washing, deglycosylation was carried out in the same Protein A cartridge, and the released glycans were eluted with wash buffer. The HPLC glycan profile obtained after labeling and cleanup, shown in Figure 3, is typical for hIgG.

### Example 2.

This study was run as in Example 1 (Figure 3), but the hIgG was spiked into a cell culture supernatant sample instead of PBS. As can be seen in Figure 4, the N-glycan profile is virtually identical as that of Figure 3.

### Example 3.

In this study, the same sample (hIgG spiked into cell culture supernatant) was run as in Example 2, but the Protein A affinity purification and deglycosylation were run using separate purification and immobilization cartridges. The results are shown in Figure 5. The resulting N-glycan profile was essentially identical to Examples 1 & 2, although the total glycan recovery is somewhat lower.

### Example 4.

This study was similar to Example 3, but the initial purification step was eliminated. The sample (hIgG spiked into culture supernatant) was denatured and loaded directly onto the immobilization cartridge for enzymatic deglycosylation. As shown in Figure 6, all of the non-IgG glycoproteins in the culture supernatant were thus present and the target hIgG is hidden in the resulting N-glycan profile. The results show the effectiveness of the purification step.

### Example 5.

In this study, culture supernatant with no added hIgG was loaded onto a Protein A cartridge. The cartridge was washed extensively (20 bed volumes) with PBS alone, and then deglycosylation, labeling and cleanup were carried out. The results are shown in Figure 7. The HPLC chromatogram is on the same scale as Figures 3 - 6. As expected, there was a very low amount of glycan showing up on the baseline, but there are still a few small peaks visible due to non-specifically adsorbed glycoproteins from the cell culture supernatant that are not effectively washed off the Protein A cartridge. These low level peaks might interfere with some analytical methods.

### Example 6.

This study duplicated Example 5, but the wash buffer had the non-ionic detergent NP-40 added. Figure 8 is an HPLC chromatogram showing the results. As shown in Figure 8, the background N-glycans from the sample matrix were reduced to non-detectable levels by the addition of detergent to the wash.

It is understood that the examples and embodiments described herein are for illustrative purposes only and that various modifications or changes in light thereof will be suggested to persons skilled in the art.

## Claims

1. A method of releasing glycans from a target glycoprotein in a biological sample, said method comprising performing the following steps in the following order:
(a) adding said biological sample to a solid phase support comprising (i) a solid phase and (ii) an affinity ligand specific for the glycoprotein, which affinity ligand is immobilized on said solid phase, thereby binding said target glycoprotein to said affinity ligand on said solid phase;
(b) washing said solid phase with a buffer to wash away any unbound glycoprotein;
and,
(c) contacting said bound target glycoprotein on said solid phase with an enzyme that releases glycans from glycoproteins,
thereby releasing said glycans from said target glycoprotein.

2. The method of claim 1, further wherein said buffer comprises a detergent, wherein optionally said detergent is a non-ionic detergent.

3. The method of claim 1 or claim 2, further wherein solid phase support is a porous chromatography media, a microcolumn or a microcartridge.

4. The method of claim 3, wherein said media is in the form of a packed bed, a monolith or a membrane.

5. The method of any preceding claim, further wherein said bound target glycoprotein is eluted from said solid phase support and quantitated, wherein optionally said quantitation is by measuring absorbance of ultraviolet light at approximately 280 nm.

6. The method of any preceding claim, optionally including step (a'), adding a blocking reagent, between step (a) and step (b).

7. A method of releasing glycans from a target glycoprotein in a biological sample, said method comprising performing the following steps in the following order:
(a) adding said biological sample to a solid phase support comprising (i) a solid phase and (ii) an affinity ligand specific for the glycoprotein, which affinity ligand is immobilized on said solid phase, thereby binding said target glycoprotein to said affinity ligand on said solid phase;
(b) washing said bound target glycoprotein with a wash buffer to wash away any unbound glycoprotein;
(c) releasing said bound target glycoprotein, and eluting said target glycoprotein from said solid phase support;
(d) optionally, quantitating the amount of said target glycoprotein eluted from said solid phase support; and
(e) contacting said target glycoprotein with an enzyme that releases glycans from glycoproteins,
thereby releasing said glycans from said target glycoprotein.

8. The method of claim 7, further comprising between steps (c) and (e), step (d') contacting said target glycoprotein to a solid phase support having a hydrophobic surface, thereby binding said target glycoprotein to said hydrophobic surface; and, optionally, step (d"), adding a blocking agent to block further binding interactions with the hydrophobic surface.

9. The method of any preceding claim, wherein said target glycoprotein is an antibody.

10. The method of any preceding claim, wherein said affinity ligand is Protein A or a deglycosylated antibody or binding fragment thereof.

11. The method of any preceding claim, wherein said enzyme is selected from the group consisting of an amidase and an endoglycosidase.

12. The method of claim 11, wherein said amidase is PNGase F.

13. The method of any preceding claim, wherein said released glycans are quantitated.

14. The method of any of claims 7 to 13, wherein said optional quantitation is by measuring absorbance of ultraviolet light at approximately 280 nm.

15. The method of any of claims 7 to 14, wherein said solid phase support is a microcolumn or microcartridge.

## Patentansprüche

1. Verfahren zum Freisetzen von Glycanen aus einem Zielglycoprotein in einer biologischen Probe, wobei das Verfahren das Ausführen der folgenden Schritte in der folgenden Reihenfolge umfasst:
(a) Zugeben der biologischen Probe zu einem Festphasenträger, der Folgendes umfasst: (i) eine Festphase und (ii) einen Affinitätsliganden, der spezifisch für das Glycoprotein ist, wobei der Affinitätsligand auf der Festphase immobilisiert ist, wodurch das Zielglycoprotein an dem Affinitätsliganden auf der Festphase gebunden wird;
(b) Waschen der Festphase mit einem Puffer, um jegliches ungebundenes Glycoprotein auszuwaschen; und,
(c) Kontaktieren des gebundenen Zielglycoproteins auf der Festphase mit einem Enzym, das Glycane aus Glycoproteinen freisetzt,
wodurch die Glycane aus dem Zielglycoprotein freigesetzt werden.

2. Verfahren nach Anspruch 1, wobei der Puffer ferner ein Detergens umfasst, wobei es sich bei dem Detergens optional um ein nichtionisches Detergens handelt.

3. Verfahren nach Anspruch 1 oder Anspruch 2, wobei es sich bei dem Festphasenträger um ein poröses Chromatographiemedium, eine Mikrosäule oder eine Mikropatrone handelt.

4. Verfahren nach Anspruch 3, wobei das Medium in der Form einer Packung, eines Monoliths oder einer Membran vorliegt.

5. Verfahren nach einem der vorstehenden Ansprüche, wobei ferner das gebundene Zielglycoprotein aus dem Festphasenträger eluiert und quantifiziert wird, wobei optional die Quantifizierung durch das Messen der Absorbanz von ultraviolettem Licht bei ungefähr 280 nm erfolgt.

6. Verfahren nach einem der vorstehenden Ansprüche, wobei dieses optional Schritt (a'), Hinzufügen eines Blockierungsreagenz, zwischen Schritt (a) und Schritt (b) beinhaltet.

7. Verfahren zum Freisetzen von Glycanen aus einem Zielglycoprotein in einer biologischen Probe, wobei das Verfahren das Ausführen der folgenden Schritte in der folgenden Reihenfolge umfasst:
(a) Zugeben der biologischen Probe zu einem Festphasenträger, der Folgendes umfasst: (i) eine Festphase und (ii) einen Affinitätsliganden, der spezifisch für das Glycoprotein ist, wobei der Affinitätsligand auf der Festphase immobilisiert ist, wodurch das Zielglycoprotein an den Affinitätsliganden auf der Festphase gebunden wird;
(b) Waschen des gebundenen Zielglycoproteins mit einem Waschpuffer, um jegliches ungebundenes Glycoprotein auszuwaschen;
(c) Freisetzen des gebundenen Zielglycoproteins und Eluieren des Zielglycoproteins aus dem Festphasenträger;
(d) optional Quantifizieren der Menge des aus dem Festphasenträger eluierten Zielglycoproteins; und
(e) Kontaktieren des Zielglycoproteins mit einem Enzym, das Glycane aus Glycoproteinen freisetzt,
wodurch die Glycane aus dem Zielglycoprotein freigesetzt werden.

8. Verfahren nach Anspruch 7, das zwischen den Schritten (c) und (e) ferner Schritt (d') Kontaktieren des Zielglycoproteins mit einem Festphasenträger, der eine hydrophobe Oberfläche aufweist, und dadurch Binden des Zielglycoproteins an diese hydrophobe Oberfläche; und optional Schritt (d") Zugeben eines Blockierungsmittels, um weitere Bindungsinteraktionen mit der hydrophoben Oberfläche zu blockieren, umfasst.

9. Verfahren nach einem der vorstehenden Ansprüche, wobei es sich bei dem Zielglycoprotein um einen Antikörper handelt.

10. Verfahren nach einem der vorstehenden Ansprüche, wobei es sich bei dem Affinitätsligand um Protein A oder einen deglycosylierten Antikörper oder ein Bindungsfragment davon handelt.

11. Verfahren nach einem der vorstehenden Ansprüche, wobei das Enzym ausgewählt ist aus der Gruppe, die aus einer Amidase und einer Endoglycosidase besteht.

12. Verfahren nach Anspruch 11, wobei es sich bei der Amidase um PNGase F handelt.

13. Verfahren nach einem der vorstehenden Ansprüche, wobei die freigesetzten Glycane quantifiziert werden.

14. Verfahren nach einem der Ansprüche 7 bis 13, wobei die optionale Quantifizierung durch Messen der Absorbanz von ultraviolettem Licht bei ungefähr 280 nm erfolgt.

15. Verfahren nach einem der Ansprüche 7 bis 14, wobei es sich bei dem Festphasenträger um eine Mikrosäule oder eine Mikropatrone handelt.

## Revendications

1. Procédé de libération de glycanes à partir d'une glycoprotéine cible dans un échantillon biologique, ledit procédé comprenant la réalisation des étapes suivantes dans l'ordre suivant :
(a) ajout dudit échantillon biologique à un support en phase solide comprenant (i) une phase solide et (ii) un ligand d'affinité spécifique de la glycoprotéine, lequel ligand d'affinité est immobilisé sur ladite phase solide, liant ainsi ladite glycoprotéine cible audit ligand d'affinité sur ladite phase solide ;
(b) lavage de ladite phase solide avec un tampon pour éliminer par lavage toute glycoprotéine non liée ; et,
(c) mise en contact de ladite glycoprotéine cible liée sur ladite phase solide avec une enzyme qui libère les glycanes des glycoprotéines,
libérant ainsi lesdits glycanes de ladite glycoprotéine cible.

2. Procédé de la revendication 1, dans lequel en outre ledit tampon comprend un détergent, ledit détergent étant facultativement un détergent non ionique.

3. Procédé de la revendication 1 ou de la revendication 2, dans lequel en outre le support en phase solide est un milieu de chromatographie poreux, une microcolonne ou une microcartouche.

4. Procédé de la revendication 3, dans lequel ledit milieu est sous la forme d'un lit fixe, d'un monolithe ou d'une membrane.

5. Procédé de l'une quelconque revendication précédente, dans lequel en outre ladite glycoprotéine cible liée est éluée dudit support en phase solide et quantifiée, dans lequel facultativement ladite quantification est par la mesure de l'absorbance de la lumière ultraviolette à environ 280 nm.

6. Procédé de l'une quelconque revendication précédente, comprenant facultativement l'étape (a'), l'addition d'un réactif de blocage, entre l'étape (a) et l'étape (b).

7. Procédé de libération de glycanes à partir d'une glycoprotéine cible dans un échantillon biologique, ledit procédé comprenant la réalisation des étapes suivantes dans l'ordre suivant :
(a) ajout dudit échantillon biologique à un support en phase solide comprenant (i) une phase solide et (ii) un ligand d'affinité spécifique de la glycoprotéine, lequel ligand d'affinité est immobilisé sur ladite phase solide, liant ainsi ladite glycoprotéine cible audit ligand d'affinité sur ladite phase solide ;
(b) lavage de ladite glycoprotéine cible liée avec un tampon de lavage pour éliminer par lavage toute glycoprotéine non liée ;
(c) libération de ladite glycoprotéine cible liée, et élution de ladite glycoprotéine cible dudit support en phase solide ;
(d) facultativement, quantification de la quantité de ladite glycoprotéine cible éluée dudit support en phase solide ; et
(c) mise en contact de ladite protéine cible avec une enzyme qui libère les glycanes des glycoprotéines,
libérant ainsi lesdits glycanes de ladite glycoprotéine cible.

8. Procédé de la revendication 7, comprenant en outre entre les étapes (c) et (e), l'étape (d') mettant en contact ladite glycoprotéine cible avec un support en phase solide ayant une surface hydrophobe, liant ainsi ladite glycoprotéine cible à ladite surface hydrophobe ; et, facultativement, une étape (d"), ajoutant un agent de blocage pour bloquer d'autres interactions de liaison avec la surface hydrophobe.

9. Procédé d'une quelconque revendication précédente, dans lequel ladite glycoprotéine cible est un anticorps.

10. Procédé d'une quelconque revendication précédente, dans lequel ledit ligand d'affinité est la Protéine A ou un anticorps déglycosylé ou un fragment de liaison de celui-ci.

11. Procédé de l'une quelconque revendication précédente, dans lequel ladite enzyme est choisie parmi le groupe constitué par une amidase et une endoglycosidase.

12. Procédé de la revendication 11, dans lequel ladite amidase est la PNGase F.

13. Procédé d'une quelconque revendication précédente, dans lequel lesdits glycanes libérés sont quantifiés.

14. Procédé de l'une quelconque des revendications 7 à 13, dans lequel ladite quantification optionnelle est par mesure de l'absorbance de la lumière ultraviolette à environ 280 nm.

15. Procédé de l'une quelconque des revendications 7 à 14, dans lequel ledit support en phase solide est une micro-colonne ou une microcartouche.
